# EUROPEAN PATENT SPECIFICATION

(11) **EP 2 182 359 B1**
(45) Date of publication and mention of the grant of the patent: **04.03.2015**
(21) Application number: 09174417.7
(22) Date of filing: 29.10.2009
(51) Int. Cl.: G01N 33/543, G01N 27/00

(54) **METHOD FOR DETECTING ANALYTE**
VERFAHREN ZUR ANALYTDETEKTION
PROCÉDÉ DE DÉTECTION D'UN ANALYTE

(30) Priority: 30.10.2008 JP 2008279385; 31.10.2008 JP 2008281584; 30.09.2009 JP 2009226321
(43) Date of publication of application: 05.05.2010
(73) Proprietor: Sysmex Corporation, Kobe-shi, Hyogo 651-0073 (JP)
(72) Inventor: Iwanaga, Shigeki, Kobe-shi Hyogo 651-0073 (JP); Kirimura, Hiroya, Kobe-shi Hyogo 651-0073 (JP)
(74) Representative: Paemen, Liesbet R.J.

(56) References cited:
- EP-A1- 1 376 111
- EP-A1- 1 947 452
- EP-A2- 0 300 651
- WO-A1-2008/007468
- WO-A2-03/062783
- WO-A2-2004/046369
- US-A1- 2002 165 675
- US-A1- 2005 053 524
- US-A1- 2007 105 119

## Description

### FIELD OF THE INVENTION

The present invention relates to a method for detecting an analyte.

### BACKGROUND

In clinical examination and diagnosis of diseases, disease-derived genes, proteins and the like contained in biological samples are detected by gene detection methods and immunological detection methods. Specific examples include immunochromatography, latex agglutination, enzyme immunoassay, chemiluminescent immunoassay, gene amplification PCR, and the like.

In these detection methods, however, there is room for improvement from the viewpoint of simplicity, rapidity, and cost.

Consequently, International Publication No. 2007/037341 has proposed a method wherein an electric current generated from a sensitizing dye by photoexcitation is utilized in detecting an analyte. In this method, a semiconductor layer is first formed on an electrode, and a probe capable of binding to the analyte is immobilized on the semiconductor layer. Then, the analyte modified with a sensitizing dye is trapped with the probe substance, and then the sensitizing dye with which the analyte is modified is irradiated with a light for exciting the sensitizing dye. As a result, electrons are emitted from the sensitizing dye with which the analyte is modified, and when the emitted electrons are received by the semiconductor layer, an electric current is generated and detected. By using a crosslinking agent such as a silane coupling agent, the probe has been immobilized on the semiconductor layer. However, the silane coupling agent has low conductivity to reduce the efficiency of detection of the electric current, and thus has a problem of low sensitivity in detection of the analyte.

In US 2002/165675 a test chip for detecting an analyte is described, comprising an electrode assembly comprising an ultra-thin metal film deposited on the surface of semiconductor material. Onto the metal film, probe molecules may be immobilized. In a multi-electrode assembly counter-electrodes are used.

### SUMMARY OF THE INVENTION

The scope of the present invention is defined solely by the appended claims, and is not affected to any degree by the statements within this summary.

The present invention relates to (1) a method for detecting an analyte modified with a modulator releasing electrons upon photoexcitation,comprising:- trapping an analyte in a sample by using a test chip, wherein the test chip comprises:
- a semiconductor electrode part including a metal layer formed on a semiconductor layer, wherein the metal layer has a thickness of between 0.2 and 10 nm, wherein when the thickness of the metal layer is 5 nm or more, the adhesion between the metal layer and the semiconductor layer is improved by using a semiconductor layer containing titanium or chrome or by using a semiconductor layer subjected to sintering;
- a probe immobilized on the metal layer, the probe trapping the analyte; and
- a counter electrode part of a conductive layer; wherein each electrode does not contact the other electrode
- modifying the analyte with a modulator;
- adding an electrolyte medium comprising iodine as an electrolyte for passing an electric current between the semiconductor electrode part and the counter electrode part and for dissolving said metal layer, thereby dissolving the metal layer;
- irradiating the modulator with light to photoexcite the modulator modifying the analyte; and
- detecting an electric current flowing generated from the photoexcited modulator.

By this constitution, a metal layer formed on a semiconductor layer can be strongly bound thereby more effectively measuring an electric current generated by photoexcitation of a modulator with which an analyte is modified, whereby the sensitivity of detection of the analyte can be increased.

According to the methods of the invention, an electric current can efficiently flow between a semiconductor electrode part and a counter electrode part, thereby increasing the efficiency of detection of an electric current generated by photoexcitation of a modulator with which an analyte is modified, whereby the sensitivity of detection of the analyte can be increased.
(2) According to certain embodiments, the probe in the method (1) of the invention for detecting an analyte is a nucleic acid, a protein or a peptide.
(3) According to certain embodiments, the metal is selected from gold, platinum, silver, palladium, nickel, mercury, rhodium, ruthenium, copper, or an alloy thereof.
(4) According to certain embodiments the metal is gold.
(5) According to certain embodiments the metal layer comprises a metal onto which the probe is chemically adsorbed.
(6) According to certain embodiments the probe has a thiol group as a bonding group for being chemically adsorbed onto the metal layer.
(7) According to certain embodiments the semiconductor layer is layer selected from silicon and germanium or a compound semiconductor or organic semiconductor selected from titanium oxide (TiO2), indium oxide (In2O2), tin oxide (SnO2), zinc oxide (ZnO), cadmium selenide (CdSe), cadmium sulfide (CdS), gallium nitride (GaN) and titanium nitride (TiN).
(8) According to certain embodiments, the counter electrode part is formed of a conductive material selected from platinum, gold, silver, copper, conductive ceramics and metal oxides.
(9) According to certain embodiments, the light source used to irradiate the modulator is selected from a laser, a light-emitting diode (LED), an inorganic electroluminescence element, an organic electroluminescence element, a white light source, and a white light source provided with an optical filter.
(10) According to certain embodiments, the metal layer has a thickness of between 0.2 nanometer and 2.0 nanometer.

### BRIEF DESCRIPTION OF THE DRAWINGS

FIG. 1 is a perspective view showing a detection apparatus 1 in accordance with one embodiment of an apparatus suitable for carrying out the methods of the present invention;
FIG. 2 is a block diagram showing the constitution of the detection apparatus 1;
FIG. 3 is a perspective view showing a test chip 4 used in the detection apparatus 1;
FIG. 4 is a perspective view showing an upper plate having a semiconductor electrode part 15 of the test chip 4;
FIG. 5 is a perspective view showing a lower plate having a counter electrode part 16 of the test chip 4;
FIG. 6 is a perspective view of the test chip 4 from which the upper substrate 13 was detached;
FIG. 7 is a sectional view showing the constitution of the test chip 4;
FIG. 8 is a schematic view showing the constitution of the semiconductor electrode part 15 and a counter electrode part 18 in the test chip 4;
FIG. 9 is a flowchart showing a method of injecting an analyte to the test chip 4 by the user;
FIG. 10 is a flowchart showing the procedure of detection operation of the detection apparatus 1;
FIG. 11 is a schematic view of the semiconductor electrode part 15 at the time of hybridization and at the time of addition of an electrolytic solution;
FIG. 12 is a graph showing photocurrent values obtained by measurement in Example 1 and Comparative Example 1;
FIG. 13 is a graph of photocurrent values detected in Example 2 and Comparative Example 2;
FIG. 14 is a graph showing a modulator-derived electric current value among data obtained in Example 2 and Comparative Example 2;
FIG. 15 is a graph of photocurrent values detected in Example 3, Comparative Example 3 and Comparative Example 4;
FIG. 16 is a graph of photocurrent values detected in Example 4, Comparative Example 5 and Comparative Example 6; and
FIG. 17 is a graph of S/N ratio in each film thickness detected in Example 5.

### DETAILED DESCRIPTION OF PARTICULAR EMBODIMENTS OF THE INVENTION

Hereinafter, the method for detecting an analyte according to the present invention will be described with reference to the drawings.

### (Constitution of the Detection Apparatus)

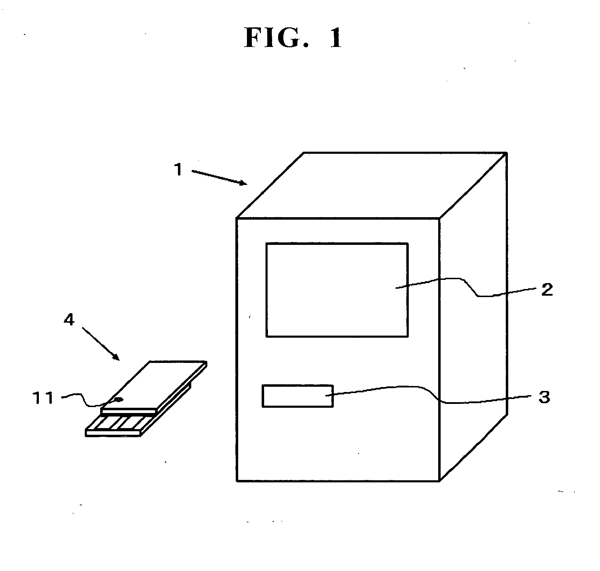

FIG. 1 is a perspective view of an embodiment of a detection apparatus suitable for carrying out the methods of the present invention. The detection apparatus is an apparatus for detecting an analyte having specific binding property, such as a nucleic acid, a protein or a peptide which was collected from living cells or synthesized artificially. The detection apparatus 1 can for example detect, in an analyte sample, mRNA of human papillomavirus (referred to hereinafter as H PV) that is a causative virus for cervical cancer.

The detection apparatus 1 includes a chip receiving part 3 into which a test chip 4 is inserted and a display 2 on which a detection result is displayed. The test chip 4 includes a sample injection port 11.

The test chip 4 is a disposable HPV detection chip and is inserted into the chip receiving part 3 of the detection apparatus 1. The test chip 4 has a function of trapping mRNA of HPV modified with a modulator generating electrons by photoexcitation upon injecting an analyte sample through the sample injection port 11.

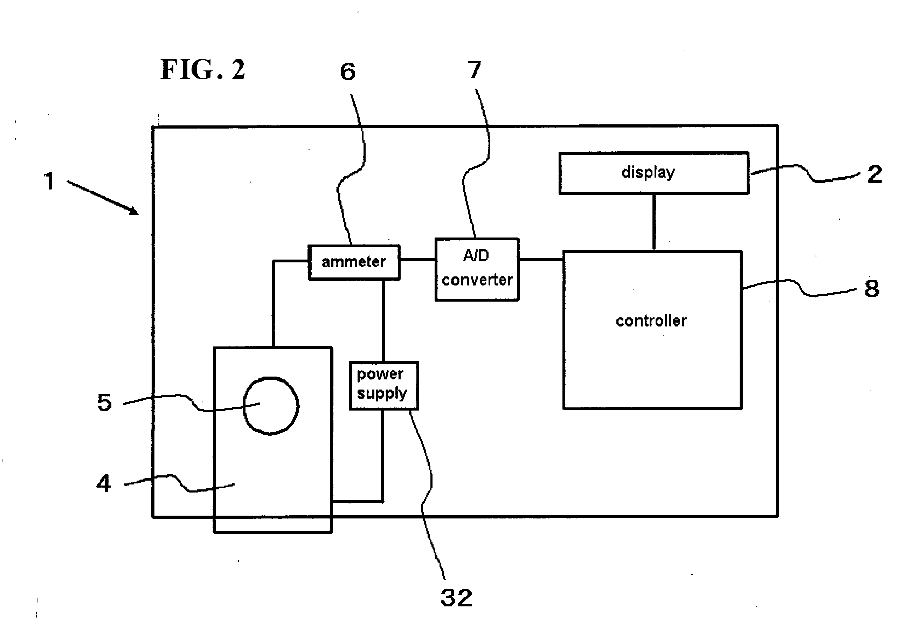

FIG. 2 is a block diagram showing the constitution of the detection apparatus 1. The detection apparatus 1 includes a light source 5, an ammeter 6, a power supply 32, an A/D converter 7, a controller 8 and a display 2.

The light source 5 applies a light to a modulator that modifies mRNA of HPV trapped by the test chip 4, thereby exciting the modulator. The ammeter 6 measures an electric current flowing due to electrons emitted from the excited modulator. The power supply 32 applies a predetermined potential to an electrode arranged in the test chip 4. The A/D converter 7 converts an electric current value measured by the ammeter 6 into a digital value. The controller 8 is composed of CPU, ROM and RAM, and regulates the operation of the light source 5, the ammeter 6, and the display 2. On the basis of a previously prepared calibration curve showing the relationship between electric current values and the amounts of HPV, the controller 8 makes a rough estimate of the amount of HPV in an analyte sample, from the digital value into which the electric current value was converted by the A/D converter 7. The display 2 displays that amount of HPV in an analyte sample which was estimated roughly by the controller 8.

### (Constitution of the Test Chip 4)

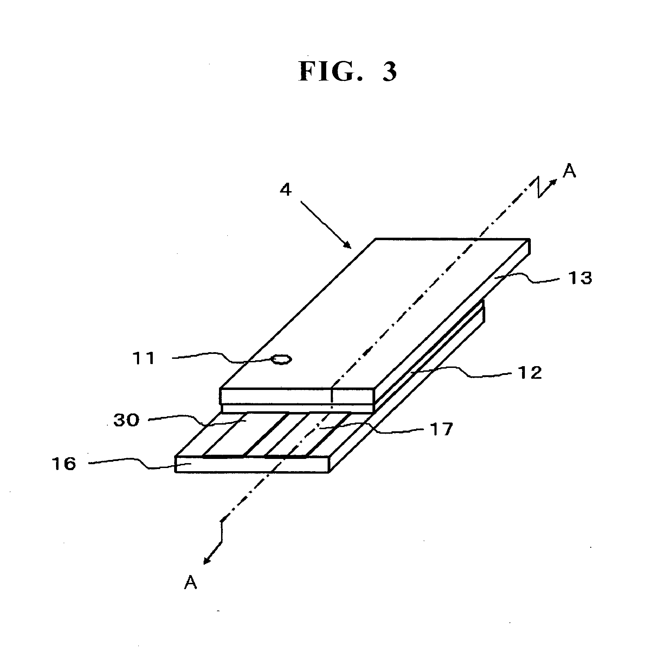

The constitution of the test chip 4 used in the detection apparatus 1 will be described with reference to FIGS. 3 to 8.

FIG. 3 is a perspective view of the test chip 4. The test chip 4 includes a lower substrate 16, an upper substrate 13 arranged above the lower substrate 16, and a silicon rubber 12 put between the lower substrate 16 and the upper substrate 13. The upper substrate 13 is provided with the sample injection port 11 communicating therein.

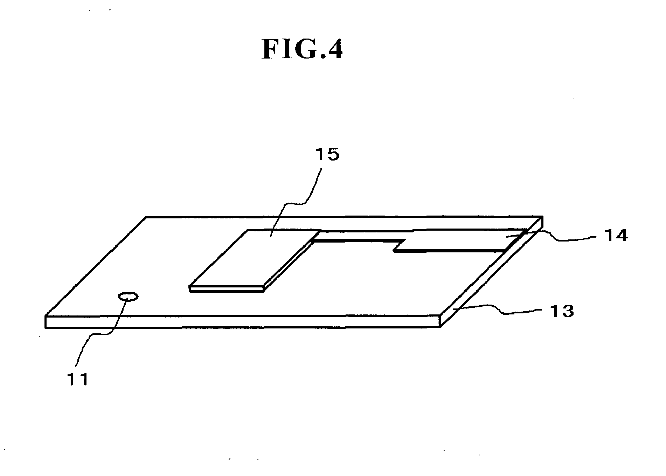

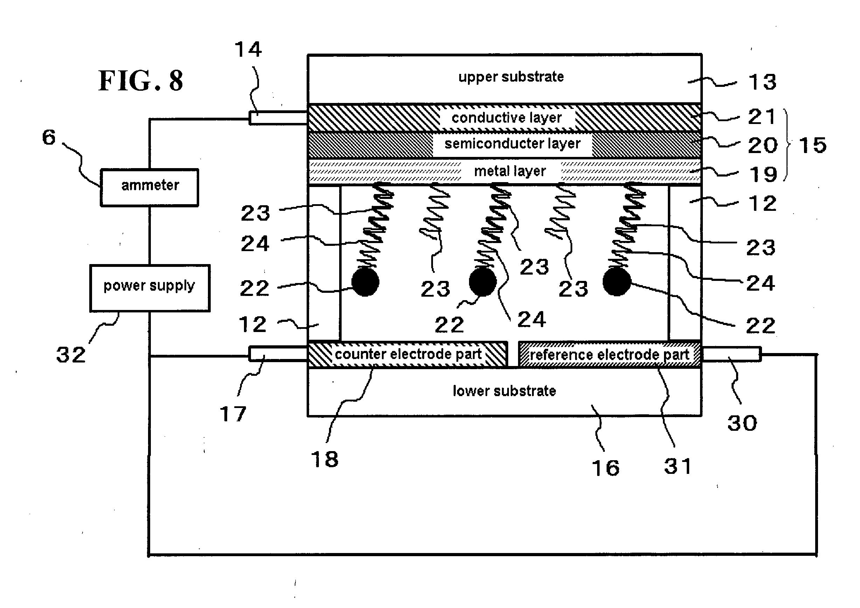

FIG. 4 is a perspective view of the upper substrate 13 after the test chip 4 in FIG. 3 is rotated horizontally right by 90° and then rotated vertically by 180°. The semiconductor electrode part 15, and an electrode lead 14 connected to the semiconductor electrode part 15, are formed on the surface of the upper substrate 13. The upper substrate 13 is formed of silicon dioxide (SiO₂), and the electrode lead 14 is formed of 2 layers consisting of indium tin oxide (ITO) and antimony-doped tin oxide (ATO). The semiconductor electrode part 15 will be described later with reference to FIG. 8.

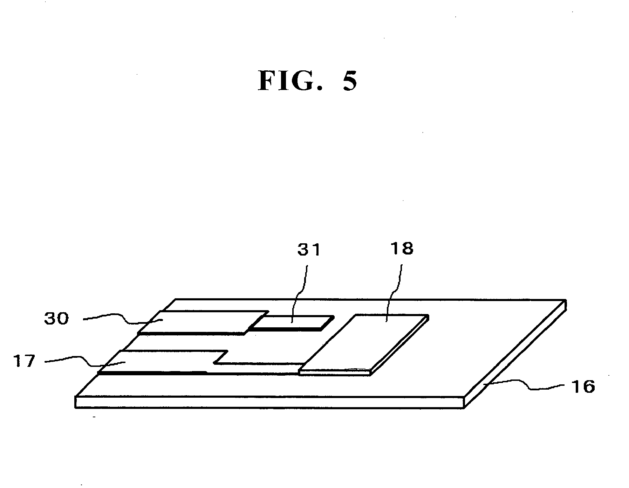

FIG. 5 is a perspective view of the lower substrate 16 after the test chip 4 in FIG. 3 is rotated horizontally right by 90°. A counter electrode part 18, an electrode lead 17 connected to the counter electrode part 18, a reference electrode 31, and an electrode lead 30 connected to the reference electrode 31, are formed on the surface of the lower substrate 16.

The lower substrate 16 is formed of glass based on silicon dioxide (SiO₂), and the counter electrode part 18, the electrode lead 17, the reference electrode 31 and the electrode lead 30 are formed of platinum respectively.

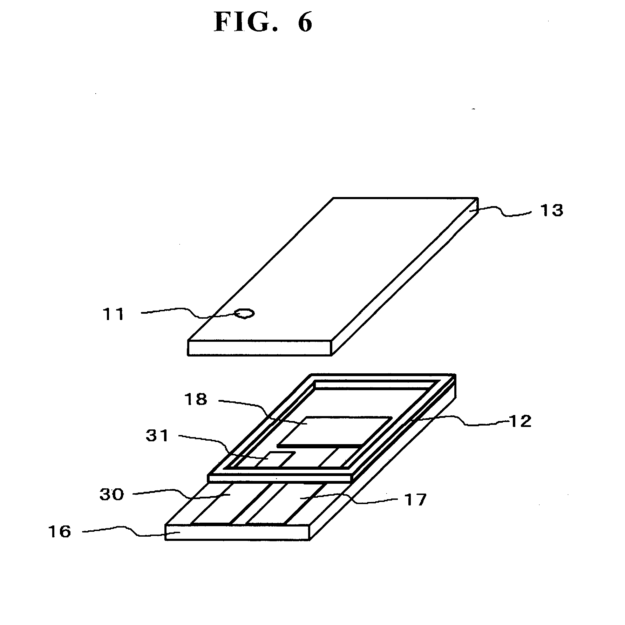

FIG. 6 is a perspective view of the test chip 4 when the upper substrate 13 of the test chip 4 in FIG. 3 is detached upwardly. The silicon rubber 12 as shown in FIG. 6 is arranged so to surround the counter electrode part 18 and the reference electrode 31 on the lower substrate 16. The electrode lead 17 connected to the counter electrode part 18 and the electrode lead 30 connected to the reference electrode 31 extend from the frame of the silicon rubber 12. The electrode lead 17 and the electrode lead 30 extending from the frame are connected to the electric power 32.

The sample injection port 11 arranged on the upper substrate 13 is a hole penetrating the upper substrate 13. An analyte sample and an electrolytic solution described later are injected through the sample injection port 11 to the frame of the silicon rubber 12.

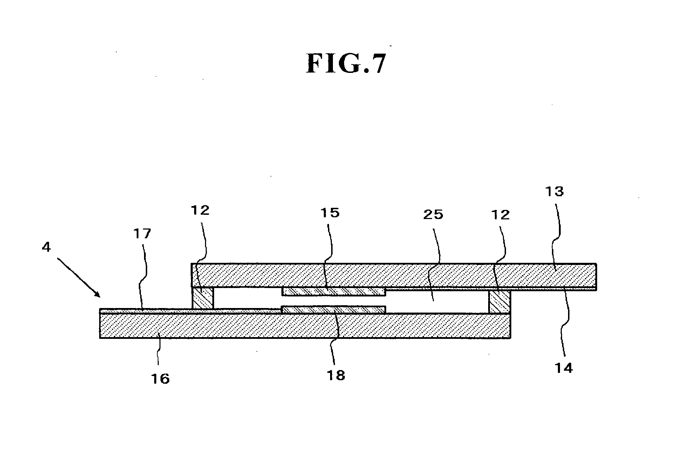

FIG. 7 is a sectional view showing an A-A sectional constitution of the test chip 4 in FIG. 3. As shown in FIG. 7, the upper substrate 13 and lower substrate 16 contained in the test chip 4 are arranged via the silicon rubber 12. A space 25 is formed between the upper substrate 13 and lower substrate 16. The semiconductor electrode part 15 formed on the upper substrate 13 is opposed via the space 25 to the counter electrode part 18 and the reference electrode part 31 (not shown) formed on the lower substrate 16. An analyte sample and an electrolytic solution described later are injected via the sample injection port 11 into the space 25.

As shown in FIG. 7, the electrode lead 14 connected to the semiconductor electrode part 15 extends along the upper substrate 13 to the outside of the space 25, and the electrode lead 17 connected to the counter electrode part 18 and the reference electrode 30 (not shown) connected to the reference electrode part 31 extend along the lower substrate 16 to the outside of the space 25. The electrode lead 14 is connected to the ammeter 6, and the electrode lead 17 and the electrode lead 30 are connected to the power supply 32.

In this embodiment, the semiconductor electrode part 15 is formed on the surface of the upper substrate 13, and the counter electrode part 18 and the reference electrode part 31 are formed on the surface of the lower substrate 16, and the arrangement between the semiconductor electrode part 15, the counter electrode part 18 and the reference electrode part 31 is not particularly limited as long as each electrode does not contact with other electrode and is arranged in the frame of the silicon rubber 12. For example, the semiconductor electrode part 15, the counter electrode part 18 and the reference electrode part 31 may be arranged on the same substrate.

The semiconductor electrode part 15 shown in FIG. 4 will be further described. FIG. 8 is a schematic diagram showing the constitution of the semiconductor electrode part 15 and counter electrode part 18.

The semiconductor electrode part 15 includes a conductive layer 21 formed on the upper substrate 13, a semiconductor layer 20 formed on the conductive layer 21, and a metal layer 19 formed on the semiconductor layer 20. The counter electrode part 18 is formed on the lower substrate 16.

A probe 23 for trapping mRNA 24 of HPV modified with a modulator 22 generating electrons by photoexcitation is fixed on the metal layer 19 contained in the semiconductor electrode part 15. The modulator 22 is a ruthenium complex and is bound, via a peptide bond, to the mRNA, thereby modifying the mRNA.

The electrode lead 14 connected to the semiconductor electrode part 15 is connected to the ammeter 6, and the electrode lead 17 connected to the counter electrode part 18 and the electrode lead 30 connected to the reference electrode part 31 are connected to the power supply 32. The ammeter 6 is connected to the power supply 32, and an electric current flowing between the semiconductor electrode part 15 and the counter electrode part 18 is measured with the ammeter 6.

The conductive layer 21 contained in the semiconductor electrode part 15 consists of 2 layers, that is, an indium tin oxide (ITO) layer formed by sputtering and antimony-doped tin oxide (ATO) formed by sputtering on the ITO layer. The semiconductor layer 20 consists of a titan oxide (TiO₂) layer formed by sputtering. The metal layer 19 consists of a gold (Au) layer formed by deposition. The counter electrode part 18 consists of a platinum layer formed by sputtering.

The probe 23 has a thiol group, and the probe 23 is immobilized on the metal layer 19 by binding a thiol group of the probe 23 to a gold atom of the metal layer 19. This immobilization is carried out by dipping the metal layer 19 in an aqueous solution having the probe 23 dispersed therein.

### (Detection Method Using HPV Detection Apparatus)

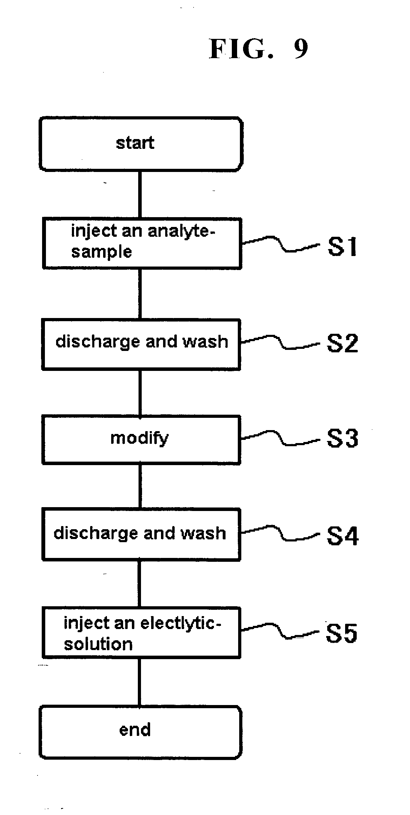

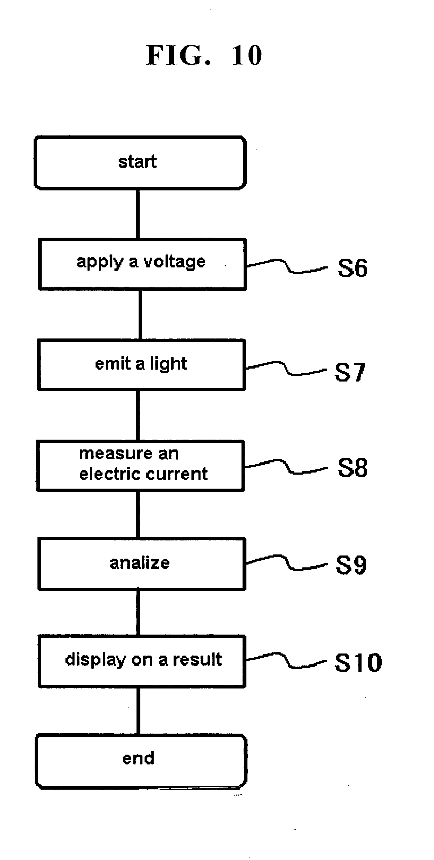

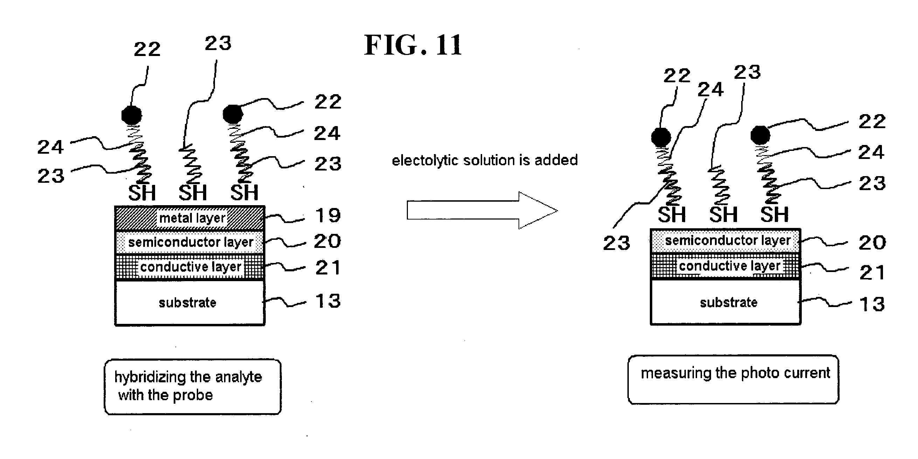

The method of using the detection apparatus 1 having the constitution described above is described with reference to FIGS. 9 to 11. FIG. 9 is a flowchart showing the procedure of injecting an analyte into the detection chip 4 by the user. FIG. 10 is a flowchart showing the procedure of detection operation of the detection apparatus 1. FIG. 11 is a schematic view of the semiconductor electrode part 15 at the time of hybridization and at the time of addition of an electrolytic solution.

From the flowchart in FIG. 9, the user in step S1 injects an analyte sample into the sample injection port 11 of the sample chip 4. This analyte sample is mRNA obtained by homogenization, extraction and purification from cervix cells. By step S1, the probe 23 on the metal layer 19 traps mRNA 24 of H PV in the analyte sample by hybridization, as shown in FIG. 11.

In step S2, the user discharges the solution in the test chip 4 from the sample injection port 11 and then washes the inside of the test chip 4 with a hybridization washing liquid.

In step S3, the user injects, through the sample injection port 11, the modulator 22 containing a nucleotide sequence capable of binding to mRNA24 of HPV. The injected modulator 22 modifies mRNA 24 trapped with the probe 23.

In step S4, the user discharges the solution in the test chip 4 from the sample injection port 11 and washes the inside of the test chip 4 with a wash buffer.

In step S5, the user injects an electrolytic solution through the sample injection port 11. This electrolyte is a mixture containing iodine as an electrolyte, tetrapropylammonium iodide as a supporting electrolyte, and an organic solvent consisting of acetonitrile and ethyl carbonate in a volume ratio of 6 : 4. When the electrolytic solution is added, the iodine contained in the electrolytic solution dissolves the metal layer 19.

The dissolution of the metal layer 19 is described with reference to FIG. 11. FIG. 11 is a schematic view of the semiconductor electrode part 15 at the time of hybridization and at the time of addition of an electrolytic solution.

The probe 23 is immobilized on the metal layer 19 by covalent bonding between a thiol group (SH group) of the probe 23 and a gold atom of the metal layer 19. The covalent bonding is a strong bonding, so that in the hybridization step (step S1) and in the washing step (step S2), the probe 23 can be prevented from releasing from the metal layer 19.

When the Electrolytic solution is added, iodine contained in the electrolytic solution dissolves the metal layer 19 consisting of gold (Au), and the probe 23 is arranged on the semiconductor layer 20. Electrons generated from the modulator 22 excited by light irradiation with the light source 5 are thereby fed efficiently to the semiconductor layer 20.

FIG. 10 is a flowchart showing the detection procedure of the detection apparatus 1. After the user performs the flow in FIG. 9, the user inserts the detection chip 4 into the chip insertion port 3 of the detection apparatus 1 shown in FIG. 1, and initiates measurement on the display 2.

In step S6, electrode leads 14, 17 and 31 of the test chip 4 inserted into the detection apparatus 1 are connected to the ammeter 6 and power supply 32. By the power supply 32, a potential of 0 V relative to the reference electrode part 31 is applied to the semiconductor electrode part 15.

In step S7, the light source 5 applies a laser light to the modulator 22 with which mRNA 24 of HPV is modified, thereby exciting the modulator 22. The excited modulator 22 releases electrons, and the released electrons are transported into the semiconductor layer 20. As a result, an electric current flows between the semiconductor electrode part 15 and the counter electrode part 18.

In step S8, the electric current which due to electron movement in step S5, flows through the semiconductor electrode part 15 and the counter electrode part 18, is measured with the ammeter 6. The electric current value measured with the ammeter 6 is correlated with the number of modulators 22, and thus HPV can be quantitatively determined on the basis of the measured electric current value.

In step S9, a digital value into which the electric current value was converted by the A/D converter 7 is inputted to the controller 8. On the basis of a previously prepared calibration curve showing the relationship between electric current values and the amounts of HPV, the controller 8 makes a rough estimate of the amount of HPV in the analyte sample, from the digital value into which the electric current value was converted. To indicate on the display 2 the roughly estimated amount of HPV, a detection result screen is formed.

Then, in step S10, the detection result screen formed by the controller 8 is transmitted to, and displayed on, the display 2.

In this embodiment, the analyte is mRNA 24 of HPV, but the analyte may be a nucleic acid, a protein or a peptide that is collected from living cells or artificially synthesized. The probe 23 may be a substance trapping an analyte, and may be for example a nucleic acid, a protein or a peptide.

Although the modulator 22 in this embodiment is a ruthenium complex, the modulator is not particularly limited as long as it is a substance to be excited by the light source 5, thereby releasing electrons. Examples of such modulators include a metal complex, an organic dye and a quantum dot. Specific examples include metal phthalocyanine, a ruthenium complex, an osmium complex, an iron complex, a zinc complex, a 9-phenylxanthene dye, a cyanine dye, a metallocyanine dye, a xanthene dye, a triphenylmethane dye, an acridine dye, an oxazine dye, a coumarin dye, a merocyanine dye, a rhodacyanine dye, a polymethine dye, a porphyrin dye, a phthalocyanine dye, a rhodamine dye, a xanthene dye, a chlorophyll dye, an eosin dye, a mercurochrome dye, an indigo dye, and a cadmium selenide dye.

In this embodiment, the light source 5 is not particularly limited as long as it emits a light with wavelength that excites the substance with which an analyte is modified. Examples of such light sources include a laser, a light-emitting diode (LED), an inorganic electroluminescence element, an organic electroluminescence element, a white light source, and a white light source provided with an optical filter.

This embodiment is illustrated wherein mRNA 24 of HPV is trapped with the probe 23 and then the mRNA 24 of HPV is modified with the modulator 22, but the mRNA 24 of HPV may be modified with the modulator 22 and then trapped with the probe 23, whereby the mRNA 24 of HPV may be detected. When the analyte and the probe are nucleic acids, there is a method of intercalation wherein a modulator is bound to a double nucleic acid formed between an analyte and a probe for trapping the analyte.

Although the metal layer 19 in this embodiment is gold, the metal layer 19 may be any metal capable of binding to the probe 23. Preferably, the metal layer 19 is a metal capable of covalently bonding to the probe 23. More preferably, the metal layer 19 is a metal capable of binding to a thiol group of the probe 23. For example, the metal layer 19 may be exemplified by gold, platinum, silver, palladium, nickel, mercury, rhodium, ruthenium, copper, or an alloy thereof. In this embodiment, the method of forming the metal layer 19 on the semiconductor layer 20 uses deposition, and may use sputtering, imprinting, screen printing, plating, or a sol-gel process.

In this embodiment, titanium oxide (TiO₂) is used as the semiconductor layer 20, but the semiconductor layer 20 may be made of a substance capable of having energy levels at which it can receive electrons released from the modulator 22 upon excitation. Examples include semiconductors such as silicon and germanium and compound semiconductors or organic semiconductors such as titanium oxide (TiO₂), indium oxide (In₂O₂), tin oxide (SnO₂), zinc oxide (ZnO), cadmium selenide (CdSe), cadmium sulfide (CdS), gallium nitride (GaN) and titanium nitride (TiN).

In this embodiment, the conductive layer 21 is formed of indium tin oxide (ITO) and antimony-doped tin oxide (ATO), but is not particularly limited as long as it is a conductive material. Examples include platinum, gold, silver and copper, and conductive ceramics and metal oxides. When the semiconductor layer 20 itself also functions as a conductive material, the conductive layer 21 can be omitted.

In this embodiment, the counter electrode part 18 is formed of platinum, but is not particularly limited as long as it is a conductive material. Examples include gold, silver and copper, and conductive ceramics and metal oxides.

In the methods according to the invention, iodine is used as a substance for dissolving the metal layer 19 and as an electrolyte.

In this embodiment, the probe 23 is bound directly to the metal layer 19, but a crosslinking agent such as ethane dithiol may be present between the probe 23 and the metal layer 19.

The detection apparatus 1 and the test chip 4 suitable for carrying out the methods of the invention, may be divided into a plurality of regions into which metal layers 19 are separated to immobilize the probe 23, whereby the light irradiation with the light source 5 may be conducted individually for each region. A plurality of samples can thereby be measured with one semiconductor electrode part 15. By immobilizing a plurality of probes on each region, many analytes and many measurement items can be measured with one test chip 4.

By the power supply 32 in this embodiment, a potential of 0 V relative to the reference electrode part 31 is applied to the semiconductor electrode part 15, but this reference electrode part 31 can be omitted. By the power supply 32 in this case, a potential of 0 V relative to the counter electrode part 18 can be applied to the semiconductor electrode part 15.

### Examples

### Example 1 (Examination of the Presence or Absence of Metal Layer on Semiconductor Electrode)

### (Preparation of Semiconductor Electrode Part)

Indium tin oxide (ITO) and antimony-doped tin oxide (ATO) were formed with a thickness of 100 nm as a conductive layer by sputtering on a substrate made of silicon dioxide (SiO₂). On this conductive layer, titanium oxide (TiO₂) was formed as a semiconductor layer of 10 nm in thickness by sputtering, and a gold thin film was formed as a metal layer of 10 nm in thickness thereon. By using a semiconductor layer containing titanium or chrome, the adhesion between the gold thin film and the semiconductor layer is improved. The member containing the conductive layer, the semiconductor layer and the metal layer is referred to the semiconductor electrode part. A semiconductor electrode lead for connection to an ammeter is connected to the semiconductor electrode part.

### (Preparation of Counter Electrode Part)

A platinum thin layer formed with a thickness of 200 nm by sputtering on a substrate made of silicon dioxide (SiO₂) was used as the counter electrode part. To this counter electrode part was connected a counter lead for connection to an ammeter.

### (Immobilization of Probe Substance)

A probe consisting of a DNA (24 bases) having a thiol group is immobilized on the metal layer of the semiconductor electrode part. First, the semiconductor electrode part was dipped for 18 hours in an aqueous solution having a nucleic acid (nucleic acid concentration 10 µM) dispersed therein. Thereafter, the semiconductor electrode part was washed with ultrapure water and dried for 30 minutes. As a result, the nucleic acid is immobilized on the metal layer by binding a thiol group of the nucleic acid to a gold atom of the metal layer.

### (Preparation of Analyte)

As the analyte, a modulator-bound DNA having a nucleotide sequence complementary to the probe is prepared. As the modulator, a sensitizing dye Pulsar 650 (manufactured by Bio Search Technologies Japan) was used. This sensitizing dye is a ruthenium complex and is bound via a peptide bond to the DNA.

### (Hybridization between Analyte and Probe)

The analyte modified with the modulator is trapped with the probe on the semiconductor electrode part.

First, silicon rubber (thickness 0.2 mm) is arranged as a partition wall around the semiconductor electrode part. 10 µL of a hybridization solution is injected into the space formed by this silicon rubber. This hybridization solution is a mixture of the modulator-modified DNA (concentration 10 µM) and a hybridization buffer (Affymetrix).

Then, the silicon rubber was covered with a cover glass and subjected to hybridization such that the solution was not dried. Hybridization was carried out by leaving it at 45°C for 1 hour. After hybridization, the sample was washed with a wash buffer A (Affymetrix) and ultrapure water, and then dried with a blower.

### (Preparation of Electrolytic Solution)

A solvent consisting of acetonitrile (AN) and ethylene carbonate (EC) mixed in a volume ratio of 6 : 4 is prepared. As a supporting electrolyte salt, tetrapropylammonium iodide (NPr₄l) is dissolved in an amount of 0.6 M. As an electrolyte, iodine is dissolved in an amount of 0.06 M. The resulting solution is used as the electrolytic solution.

### (Measurement of Photocurrent)

Silicon rubber (thickness 0.2 mm) is arranged as a side wall around the substrate having the semiconductor electrode part where the analyte was hybridized with the probe. 10 µL of the electrolytic solution is injected into the space formed by this silicon rubber. Then, the semiconductor electrode part filled with the electrolytic solution is sealed from above with the substrate having the counter electrode part. The semiconductor electrode part and the counter electrode part are thereby contacted with the electrolytic solution.

Then, the semiconductor electrode lead and the counter electrode lead are connected to the ammeter. Then, the light source emits a light from the direction of the semiconductor electrode part to the counter electrode part. The light source is a laser light source having a wavelength of 473 nm and an intensity of 13 mW. As a result, the modulator is excited, electrons generated from the excited modulator are transported into the semiconductor layer, and an electric current flows through the semiconductor electrode part and the counter electrode part. This electric current value is measured.

### Comparative Example 1

Comparative Example 1 is the same as in Example 1 except that the step of forming the metal layer on the semiconductor layer is not performed.

### (Results)

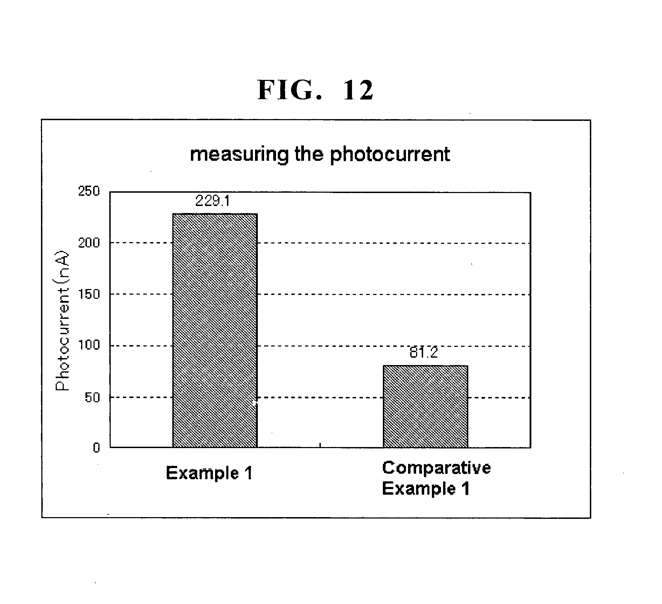

FIG. 12 is a graph showing electric current values obtained in measurement of Example 1 and Comparative Example 1. By the method in Example 1, an electric current value of 229 nA was obtained. By the method in Comparative Example 1, on the other hand, an electric current value of 80 nA was obtained.

From this result, it could be seen that by forming the metal layer on the semiconductor layer, an electric current value as high as 3 times can be extracted, and the detection sensitivity of an electric current value was improved.

### Example 2 (Detection by Current Measurement of Analyte Modified with Modulator)

### (Preparation of Semiconductor Electrode Part)

Prepared in the same manner as in Example 1.

### (Preparation of Counter Electrode)

Prepared in the same manner as in Example 1.

### (Immobilization of Probe)

Performed in the same manner as in Example 1.

### (Preparation of Analyte)

An analyte (analyte A) having a modulator bound to a DNA containing a nucleotide sequence complementary to the probe and an analyte (analyte B) having a modifier bound to a DNA not containing a nucleotide sequence complementary to the probe are prepared as analytes.

The modulator is a sensitizing dye Pulsar 650 (manufactured by Bio Search Technologies Japan). This sensitizing dye is a ruthenium complex and is bound to the DNA via a peptide bond.

### (Trap of Analyte with Probe)

The analyte A or B is subjected to hybridization reaction with the probe on the metal layer. First, silicon rubber (thickness 0.2 mm) is arranged as a partition wall around the semiconductor electrode part. 10 µL of a hybridization solution is injected into the space formed by this silicon rubber. This hybridization solution is a mixture of the modulator-modified DNA (concentration 10 µM) and a hybridization buffer (Affymetrix).

Then, the silicon rubber was covered with a cover glass and subjected to hybridization such that the solution was not dried. Hybridization is carried out by leaving it at 45°C for 1 hour. After hybridization, the sample was washed with a wash buffer A (Affymetrix) and ultrapure water, and then dried with a blower.

### (Preparation of Electrolytic Solution)

Performed in the same manner as in Example 1.

### (Measurement of Electric Current)

Silicon rubber (thickness 0.2 mm) is arranged as a side wall around the substrate having the semiconductor electrode part where the analyte A or B was hybridized with the probe. 10 µL of an electrolytic solution is injected into the space formed by this silicon rubber, and the semiconductor electrode part filled with the electrolytic solution is sealed from above with the substrate having the counter electrode part. The semiconductor electrode part and the counter electrode part are thereby contacted with the electrolytic solution.

Then, the semiconductor electrode lead and the counter electrode lead are connected to the ammeter, and the light source emits a light from the direction of the semiconductor electrode part to the counter electrode part. The light source was a laser light source having a wavelength of 473 nm and an intensity of 13 mW. The modulator with which the analyte is modified is thereby excited, electrons released from the excited modulator are transported into the semiconductor layer, and an electric current flows through the semiconductor electrode part and the counter electrode part. This electric current value was measured.

An electric current is measured as an electrode-derived electric current without conducting the step of hybridizing the analyte with the probe on the metal layer. The electrode-derived electric current refers to an electric current generated by irradiation of the electrode itself.

### Comparative Example 2

The analyte was detected in the same manner as in Example 2 except that the step of forming the metal layer on the semiconductor layer was not carried out.

### (Results)

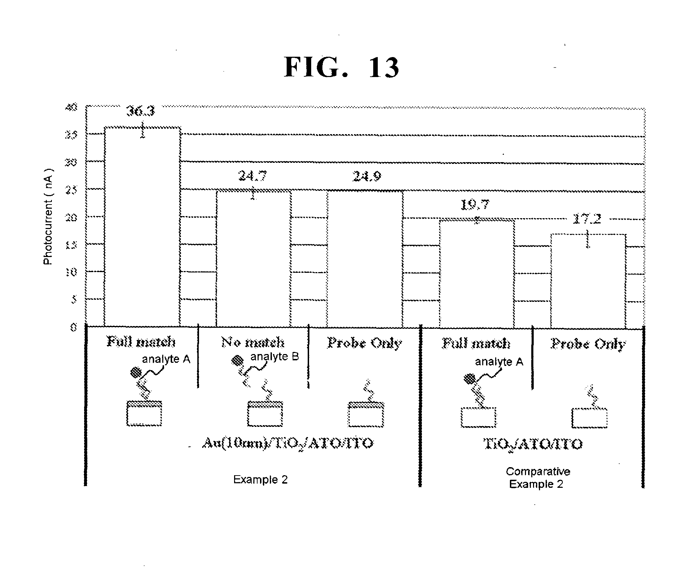

FIG. 13 is a graph of electric current values detected in Example 2 and

### Comparative Example 2.

In Example 2, the electric current value detected was 36.9 nA when hybridization was conducted with the DNA (analyte A) having a nucleotide sequence complementary to the probe.

When hybridization was conducted with the DNA (analyte B) not having a nucleotide sequence complementary to the probe, the current value detected was 24.7 nA. This electric current value was equivalent to the electrode-derived electric current value of 24.9 nA. Accordingly, it can be confirmed that the electric current value detected by hybridization with the analyte A is not due to the unspecific adsorption of the analyte onto the semiconductor electrode part but due to specific detection by recognition of the sequence.

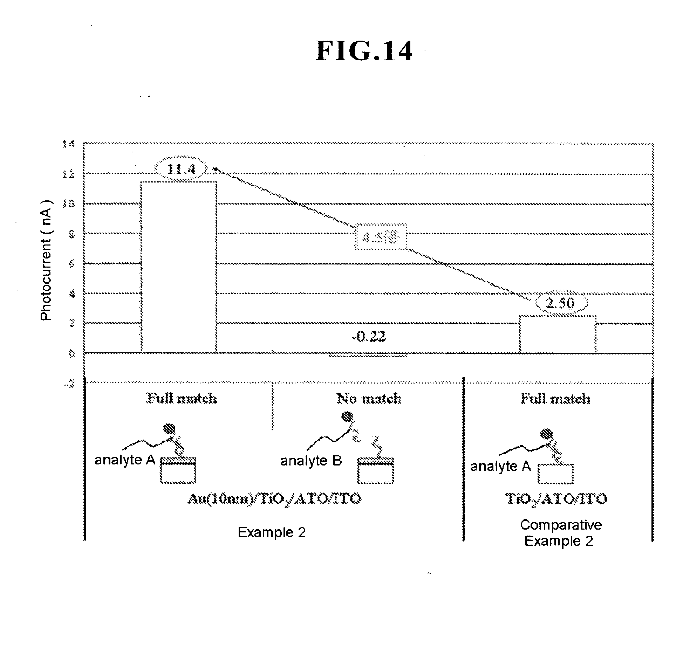

FIG. 14 is a graph showing modulator-derived electric current values among data obtained in Example 2 and Comparative Example 2. The modulator-derived electric current value refers to an electric current value obtained by subtracting an electrode-derived electric current value from an electric current value obtained by measuring the analyte.

The modulator-derived electric current value is greater by about 4.5 times in the metal layer-containing semiconductor electrode part (Example 2) than in the metal layer-free semiconductor electrode part (Comparative Example 2).

### Example 3 (Effect of the Semiconductor Electrode Using Modulator Excited with Long Wavelength)

### (Preparation of Semiconductor Electrode Part)

Indium tin oxide (ITO) was formed with a thickness of 100 nm as a conductive layer by sputtering on a substrate made of silicon dioxide (SiO₂). On this conductive layer, indium oxide (In₂O₃) was formed as a semiconductor layer of 10 nm in thickness by sputtering, and a gold thin film was formed as a metal layer of 10 nm in thickness thereon. It was calcinated (150°C) in an oxygen atmosphere, thereby improving the adhesion between the gold thin film and the semiconductor layer. The member containing the conductive layer, the semiconductor layer and the metal layer is referred to the semiconductor electrode part. A semiconductor electrode lead for connection to the ammeter is connected to the semiconductor electrode part.

### (Preparation of Counter Electrode Part)

Prepared in the same manner as in Example 1.

### (Immobilization of Probe Substance)

A probe consisting of a DNA (24 bases) having a thiol group is immobilized on the metal layer of the semiconductor electrode part. First, the semiconductor electrode part was dipped for 18 hours in an aqueous solution having a nucleic acid (nucleic acid concentration 10 µM) dispersed therein. Thereafter, the semiconductor electrode part was washed with ultrapure water and dried for 10 minutes. As a result, the nucleic acid is immobilized on the metal layer by binding a thiol group of the nucleic acid to a gold atom of the metal layer.

### (Preparation of Analyte)

As the analyte, a modulator-bound DNA having a nucleotide sequence complementary to the probe is prepared. As the modulator, Alexa Fluor 750 (Invitrogen) was used. This modulator is an organic dye and is bound via a peptide bond to the DNA.

### (Hybridization between Analyte and Probe)

The dye-modified analyte is trapped with the probe on the semiconductor electrode part.

First, silicon rubber (thickness 0.2 mm) is arranged as a partition wall around the semiconductor electrode part. 10 µL of a hybridization solution is injected into the space formed by this silicon rubber. This hybridization solution was a mixture of the modulator-modified DNA (concentration 10 µM) and a hybridization buffer (Affymetrix).

Then, the silicon rubber was covered with a cover glass and subjected to hybridization such that the solution was not dried. Hybridization is carried out by leaving it at 45°C for 1 hour. After hybridization, the sample was washed with a wash buffer A (Affymetrix) and ultrapure water, and then dried with a blower.

### (Preparation of Electrolytic solution)

First, a solvent consisting of acetonitrile (AN) and ethylene carbonate (EC) mixed in a volume ratio of 6 : 4 is prepared. As a supporting electrolyte salt, tetrapropylammonium iodide (NPr₄l) is dissolved in an amount of 0.6 M. As an electrolyte, iodine is dissolved in an amount of 0.06 M. The resulting solution is used as the electrolytic solution.

### (Measurement of Photocurrent)

Silicon rubber (thickness 0.2 mm) is arranged to surround the substrate having the semiconductor electrode where the analyte was hybridized with the probe. 10 µL of the electrolytic solution is injected into the space formed by this silicon rubber. The semiconductor electrode part filled with the electrolytic solution is sealed from above with the substrate having the counter electrode part. The semiconductor electrode part, the counter electrode part and the reference electrode part are thereby contacted with the electrolytic solution.

Then, a voltage of 0 V relative to the reference electrode is applied to the semiconductor electrode. Then, the light source emits a light from the direction of the semiconductor electrode part to the counter electrode part. The light source was a laser light source (Cube 785, Coherent Ltd.) having a wavelength of 785 nm and an intensity of 13 mW. The modulator is thereby excited, electrons released from the excited modulator are transported into the semiconductor layer, and an electric current flows through the semiconductor electrode part and the counter electrode part. This electric current value was measured.

An electric current is measured as an electrode-derived electric current without conducting the step of hybridizing the analyte with the probe on the metal layer. The electrode-derived electric current refers to an electric current generated by irradiation of the electrode itself.

### Comparative Example 3

In Comparative Example 3, the same operation as in Example 3 was conducted except that the step of forming the metal layer on the semiconductor layer was not carried out.

### Comparative Example 4

In Comparative Example 4, a silane coupling agent (aminopropyltriethoxysilane: APTES) was used in immobilizing the probe DNA.

### (Preparation of Semiconductor Electrode Part)

Indium tin oxide (ITO) was formed with a thickness of 100 nm as a conductive layer by sputtering on a substrate made of silicon dioxide (SiO₂). On this conductive layer, indium oxide (In₂O₃) was formed as a semiconductor layer of 10 nm in thickness by sputtering. This electrode was dipped in a solution having a silane coupling agent (aminopropyltriethoxysilane: APTES) dissolved at a concentration of 1% in toluene, to form a thin film of the silane coupling agent on the semiconductor layer. Then, the electrode was heated at 110°C, then washed with sonication (5 minutes) 3 times in toluene, and washed with dehydrated ethanol, thereby removing the silane coupling agent not bound to the surface of the semiconductor electrode. This conductive layer and the semiconductor layer serve as the semiconductor electrode part. To the semiconductor electrode part was connected the semiconductor electrode lead for connection to an ammeter.

### (Preparation of Counter Electrode Part)

Prepared in the same manner as in Example 3.

### (Immobilization of Probe Substance)

A probe consisting of a DNA (24 bases) is immobilized on the semiconductor layer. First, 6 µL of a solution in which an aqueous solution having a nucleic acid (nucleic acid concentration 100 µM) dispersed therein and an UV crosslinking reagent (Microarray crosslinking reagent D, Amersham) were mixed in a mixing ratio of 1 : 9 was dropped onto the semiconductor electrode. Thereafter, the resultant was irradiated with UV light (160 mJ) with UV crosslinker (FS-1500, Funakoshi), then washed with ultrapure water and dried for 10 minutes.

As a result, the UV crosslinking reagent serves as a crosslinking agent between the DNA and the silane coupling agent, and the nucleic acid is immobilized on the semiconductor layer.

### (Preparation of Analyte)

Prepared in the same manner as in Example 3.

### (Hybridization between Analyte and Probe)

Performed in the same manner as Example 3.

### (Preparation of Electrolytic Solution)

Prepared in the same manner as Example 3.

### (Measurement of Photocurrent)

Performed in the same manner as in Example 3.

### (Results)

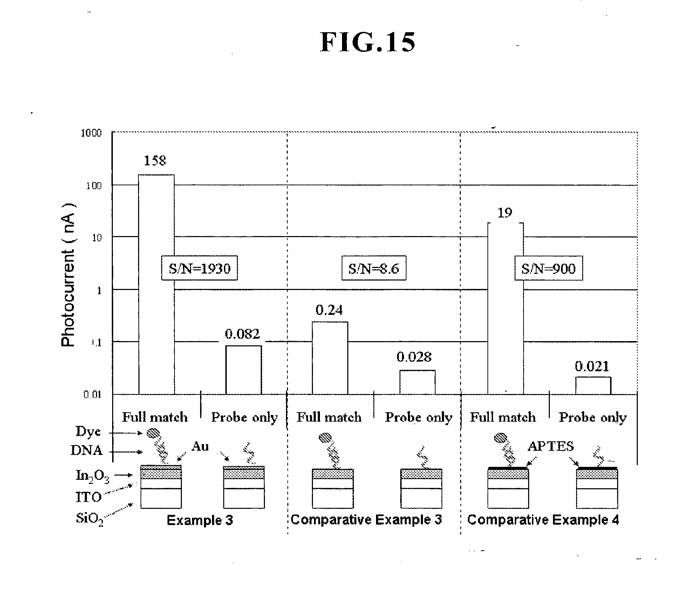

FIG. 15 is a graph of photocurrent values detected in Example 3, Comparative Example 3 and Comparative Example 4.

In Example 3, the photocurrent value detected was 158 nA when hybridization was conducted with the DNA having a nucleotide sequence complementary to the probe. When the probe DNA only was immobilized, the photocurrent value detected was 0.082 nA. From this result, S/N = 158/0.082 = 1930.

In Comparative Example 3, the photocurrent value detected was 0.24 nA when hybridization was conducted with the DNA having a nucleotide sequence complementary to the probe. When the probe DNA only was immobilized, the photocurrent value detected was 0.028 nA. From this result, S/N = 0.24/0.028 = 8.6. When compared with Example 3, it is revealed that the modulator-derived photocurrent value was 660 times and the S/N ratio was 220 times.

In Comparative Example 4, the photocurrent value detected was 19 nA when hybridization was conducted with the DNA having a nucleotide sequence complementary to the probe. When the probe DNA only was immobilized, the photocurrent value detected was 0.021 nA. From this result, S/N =19/0.021 = 900. When compared with Example 3, it is revealed that the modulator-derived photocurrent value was 8 times and the S/N ratio was 2 times. Similarly to Comparative Example 4, an improvement in modulator-derived photocurrent value and an improvement in S/N ratio are observed.

From the foregoing, it is revealed that when a metal layer is formed on the semiconductor electrode part, the detection sensitivity of electric current is improved. The estimated factor for improvement in detection sensitivity of electric current values is that by forming the metal layer, there is brought about (1) increase in the amount of DNA immobilized, (2) improvement in conductivity, and (3) improvement in photoelectric conversion by plasmon excitation in the metal layer.

### Example 4 (Verification of Nonspecific Adsorption by Single Nucleotide Polymorphism (SNP))

### (Preparation of Semiconductor Electrode Part)

Indium tin oxide (ITO) was formed with a thickness of 100 nm as a conductive layer by sputtering on a substrate made of silicon dioxide (SiO₂). On this conductive layer, indium oxide (In₂O₃) was formed as a semiconductor layer of 10 nm in thickness by sputtering, and a gold thin film was formed as a metal layer of 2 nm in thickness thereon by deposition. The member containing the conductive layer, the semiconductor layer and the metal layer is referred to the semiconductor electrode part. A semiconductor electrode lead for connection to the ammeter is connected to the semiconductor electrode part.

### (Preparation of Counter Electrode Part)

A platinum thin layer formed with a thickness of 200 nm by sputtering on a substrate made of silicon dioxide (SiO₂) was used as the counter electrode part. To this counter electrode part was connected a counter lead for connection to the ammeter.

### (Immobilization of Probe Substance)

A probe consisting of a DNA (24 bases) having a thiol group is immobilized on the metal layer of the semiconductor electrode part. First, the semiconductor electrode part is dipped for 18 hours in an aqueous solution having a nucleic acid (nucleic acid concentration 10 µM) dispersed therein. Thereafter, the semiconductor electrode part is washed with ultrapure water and dried for 10 minutes. As a result, the nucleic acid is immobilized on the metal layer by binding a thiol group of the nucleic acid to a gold atom of the metal layer.

### (Preparation of Analyte)

An analyte having a modulator bound to a DNA containing a non-complementary nucleotide sequence (with only 1 non-complementary base) to the probe is prepared. The modulator is Alexa Fluor 750 (Invitrogen). This modulator is an organic dye and is bound to the DNA via a peptide bond.

### (Hybridization between Analyte and Probe)

The analyte modified with the dye is trapped with the probe on the semiconductor electrode part.

First, silicon rubber (thickness 0.2 mm) is arranged as a partition wall around the semiconductor electrode part. 10 µL of a hybridization solution is injected into the space made of this silicon rubber. This hybridization solution was a mixture of the modulator-modified DNA (concentration 10 µM) and a hybridization buffer (Affymetrix).

Then, the silicon rubber was covered with a cover glass and subjected to hybridization such that the solution was not dried. Hybridization was carried out by leaving it at 45°C for 1 hour. After hybridization, the sample was washed with a wash buffer A (Affymetrix) and ultrapure water, and then dried with a blower.

### (Preparation of Electrolytic Solution)

A solvent consisting of acetonitrile (AN) and ethylene carbonate (EC) mixed in a volume ratio of 6 : 4 is prepared. As a supporting electrolyte salt, tetrapropylammonium iodide (NPr₄I) is dissolved in an amount of 0.6 M. As an electrolyte, iodine is dissolved in an amount of 0.06 M. The resulting solution is used as the electrolytic solution.

### (Measurement of Photocurrent)

Silicon rubber (thickness 0.2 mm) is arranged as a side wall around the substrate having the semiconductor electrode part where the analyte was hybridized with the probe. 10 µL of the electrolytic solution is injected into the space formed by this silicon rubber. Then, the semiconductor electrode part filled with the electrolytic solution is sealed from above with the substrate having the counter electrode part. The semiconductor electrode part and the counter electrode part are thereby contacted with the electrolytic solution.

Then, a voltage of 0 V relative to the reference electrode is applied to the semiconductor electrode. Then, the light source emits a light from the direction of the semiconductor electrode part to the counter electrode part. The light source was a laser light source (Cube 785, Coherent Ltd.) having a wavelength of 785 nm and an intensity of 13 mW. As a result, the modulator is excited, electrons released from the excited modulator are transported into the semiconductor layer, and an electric current flows through the semiconductor electrode part and the counter electrode part. This electric current value was measured.

### Comparative Example 5

In Comparative Example 5, the same operation as in Example 4 was conducted except that a nucleotide sequence complementary to the probe was used.

### Comparative Example 6

In Comparative Example 6, the same operation as in Example 4 was conducted except that the analyte was not hybridized.

### (Results)

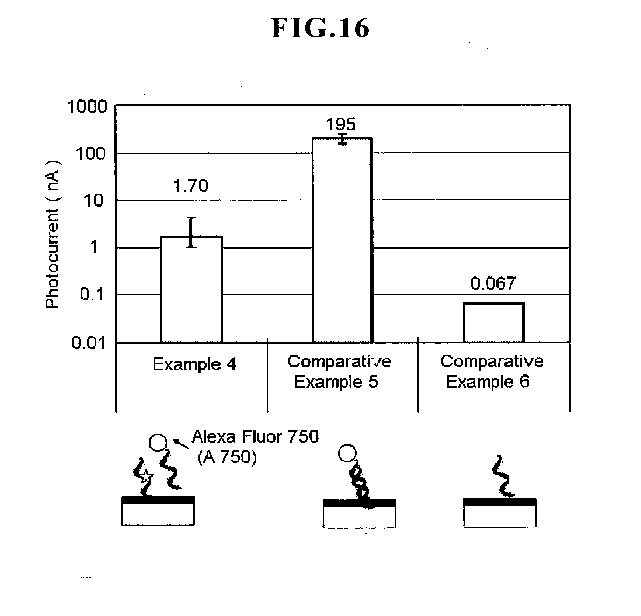

FIG. 16 is a graph of photocurrent values detected in Example 4, Comparative Example 5 and Comparative Example 6.

In Example 4, the photocurrent value detected was 1.7 nA when hybridization reaction was conducted with the DNA having a non-complementary nucleotide sequence to the probe. In Comparative Example 5, the photocurrent value detected was 195 nA when hybridization reaction was conducted with the DNA having a complementary nucleotide sequence to the probe. When the probe DNA only was immobilized in Comparative Example 6, the photocurrent value detected was 0.067 nA. From this result, it can be confirmed that the amount of DNA adsorbing nonspecifically into the gold thin film is small, and the analyte is detected sequence-specifically.

### Example 5 (Dependence of Gold Thin Film on Film Thickness)

### (Preparation of Semiconductor Electrode Part)

Indium tin oxide (ITO) was formed with a thickness of 100 nm as a conductive layer by sputtering on a substrate made of silicon dioxide (SiO₂). On this conductive layer, indium oxide (In₂O₃) was formed as a semiconductor layer of 10 nm in thickness by sputtering, and a gold thin film was formed as a metal layer of 10 nm in thickness thereon by deposition. The member containing the conductive layer, the semiconductor layer and the metal layer is referred to the semiconductor electrode part. A semiconductor electrode lead for connection to the ammeter is connected to the semiconductor electrode part.

### (Preparation of Counter Electrode Part)

A platinum thin layer was formed with a thickness of 200 nm as a conductive layer by sputtering on a substrate made of silicon dioxide (SiO₂), and the resultant was used as the counter electrode part. To this counter electrode part was connected a counter electrode lead for connection to the ammeter.

### (Immobilization of Probe Substance)

A probe consisting of a DNA (24 bases) having a thiol group is immobilized on the metal layer of the semiconductor electrode part. First, the semiconductor electrode part is dipped for 16 hours in an aqueous solution having a nucleic acid (nucleic acid concentration 10 µM) dispersed therein. Thereafter, the semiconductor electrode part is washed with ultrapure water and dried for 10 minutes. As a result, the nucleic acid is immobilized on the metal layer by binding a thiol group of the nucleic acid to a gold atom of the metal layer.

### (Preparation of Analyte)

An analyte having a modulator bound to a DNA containing a complementary nucleotide sequence to the probe is prepared. The modulator is Alexa Fluor 750 (Invitrogen). This modulator is an organic dye and is bound to the DNA via a peptide bond.

### (Hybridization between Analyte and Probe)

The analyte modified with the dye is trapped with the probe on the semiconductor electrode part.

First, silicon rubber (thickness 0.2 mm) is arranged as a partition wall around the semiconductor electrode part. 10 µL of a hybridization solution is injected into the space made of this silicon rubber. This hybridization solution was a mixture of the modulator-modified DNA (concentration 10 µM) and a hybridization buffer (Affymetrix).

Then, the silicon rubber was covered with a cover glass and subjected to hybridization such that the solution was not dried. Hybridization was carried out by leaving it at 45°C for 1 hour. After hybridization, the sample was washed with a wash buffer A (Affymetrix) and ultrapure water, and then dried with a blower.

### (Preparation of Electrolytic Solution)

A solvent consisting of acetonitrile (AN) and ethylene carbonate (EC) mixed in a volume ratio of 6 : 4 is prepared. As a supporting electrolyte salt; tetrapropylammonium iodide (NPr₄l) is dissolved in an amount of 0.6 M. As an electrolyte, iodine is dissolved in an amount of 0.06 M. The resulting solution is used as the electrolytic solution.

### (Measurement of Photocurrent)

Silicon rubber (thickness 0.2 mm) is arranged as a side wall around the substrate having the semiconductor electrode part where the analyte was hybridized with the probe. 10 µL of the electrolytic solution is injected into the space formed by this silicon rubber. Then, the semiconductor electrode part filled with the electrolytic solution is sealed from above with the substrate having the counter electrode part. The semiconductor electrode part and the counter electrode part are thereby contacted with the electrolytic solution.

Then, a voltage of 0 V relative to the reference electrode is applied to the semiconductor electrode. Then, the light source emits a light from the direction of the semiconductor electrode part to the counter electrode part. The light source was a laser light source (Cube 785, Coherent Ltd.) having a wavelength of 785 nm and an intensity of 13 mW. As a result, the modulator is excited, electrons released from the excited modulator are transported into the semiconductor layer, and an electric current flows through the semiconductor electrode part and the counter electrode part. This electric current value was measured.

When the thickness of the gold thin film was changed to 1 nm, 2 nm or 5 nm in preparation of the semiconductor electrode part, electric current values were measured in the same manner as described above.

An electric current was measured as an electrode-derived current without conducting the step of hybridizing the analyte with the probe on the metal layer. The electrode-derived current refers to an electric current generated by irradiation of the electrode itself.

### (Results)

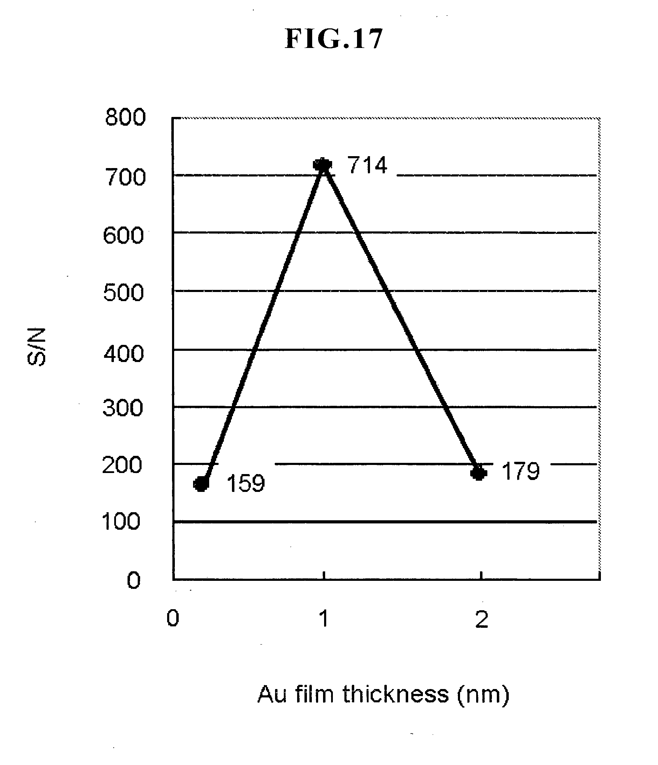

FIG. 17 is a graph of S/N ratio in each film thickness detected in Example 5. It can be seen that when the gold thin film is 1 nm, the best S/N ratio can be obtained.

When the gold thin film was 5 nm or more, the gold thin film was released from the semiconductor layer by a washing step. Accordingly, when the thickness of the gold thin film was 5 nm or more, the adhesion between the gold thin film and the semiconductor layer should be improved by using a semiconductor layer containing titanium or chrome or by using a semiconductor layer subjected to sintering.

## Claims

1. A method for detecting an analyte modified with a modulator releasing electrons upon photoexcitation, comprising:
- trapping an analyte in a sample by using a test chip, wherein the test chip comprises:
- a semiconductor electrode part including a metal layer formed on a semiconductor layer, wherein the metal layer has a thickness of between 0.2 and 10 nm, wherein when the thickness of the metal layer is 5 nm or more, the adhesion between the metal layer and the semiconductor layer is improved by using a semiconductor layer containing titanium or chrome or by using a semiconductor layer subjected to sintering;
- a probe immobilized on the metal layer, the probe trapping the analyte; and
- a counter electrode part of a conductive layer; wherein each electrode does not contact the other electrode
- modifying the analyte with a modulator;
- adding an electrolyte medium comprising iodine as an electrolyte for passing an electric current between the semiconductor electrode part and the counter electrode part and for dissolving said metal layer, thereby dissolving the metal layer;
- irradiating the modulator with light to photoexcite the modulator modifying the analyte; and
- detecting an electric current flowing generated from the photoexcited modulator.

2. The method of claim 1, wherein the probe is a nucleic acid, a protein or a peptide.

3. The method according to claim 1 or 2, wherein the metal is selected from gold, platinum, silver, palladium, nickel, mercury, rhodium, ruthenium, copper, or an alloy thereof.

4. The method of claim 3, wherein the metal is gold.

5. The method of any one of claims 1 to 4, wherein the metal layer comprises a metal onto which the probe is chemically adsorbed.

6. The method of any one of claims 1 to 5, wherein the probe has a thiol group as a bonding group for being chemically adsorbed onto the metal layer.

7. The method according to any one of claims 1 to 6, wherein the semiconductor layer is layer selected from silicon and germanium or a compound semiconductor or organic semiconductor selected from titanium oxide (TiO₂), indium oxide (In₂O₂), tin oxide (SnO₂), zinc oxide (ZnO), cadmium selenide (CdSe), cadmium sulfide (CdS), gallium nitride (GaN) and titanium nitride (TiN).

8. The method according to any one of claims 1 to 7, wherein the counter electrode part is formed of a conductive material selected from platinum, gold, silver, copper, conductive ceramics and metal oxides.

9. The method according to any one of claims 1 to 8, wherein the light source used to irradiate the modulator is selected from a laser, a light-emitting diode (LED), an inorganic electroluminescence element, an organic electroluminescence element, a white light source, and a white light source provided with an optical filter.

10. The method according to any one of claim 1 to 9, wherein the metal layer has a thickness of between 0.2 nanometer and 2.0 nanometer.

## Patentansprüche

1. Verfahren zum Detektieren eines Analyten, der mit einem Modulator modifiziert ist, der bei fotochemischer Anregung Elektronen freisetzt, das Folgendes umfasst:
- Einfangen eines Analyten in einer Probe mittels eines Testchips, wobei der Testchip Folgendes umfasst:
- einen Halbleiterelektrodenteil mit einer auf einer Halbleiterschicht gebildeten Metallschicht, wobei die Metallschicht eine Dicke zwischen 0,2 und 10 nm aufweist, wobei, wenn die Dicke der Metallschicht 5 nm oder mehr beträgt, die Adhäsion zwischen der Metallschicht und der Halbleiterschicht durch Verwendung einer Halbleiterschicht, die Titan oder Chrom enthält, oder durch Verwendung einer Halbleiterschicht, die gesintert wird, verbessert wird;
- eine auf der Metallschicht immobilisierte Sonde, wobei die Sonde den Analyten einfängt; und
- einem Gegenelektrodenteil einer leitfähigen Schicht; wobei jede Elektrode nicht mit der anderen Elektrode in Kontakt steht;
- Modifizieren des Analyten mit einem Modulator;
- Hinzufügen eines Elektrolytmediums, das Jod als Elektrolyt umfasst, um einen elektrischen Strom zwischen dem Halbleiterelektrodenteil und dem Gegenelektrodenteil fließen zu lassen und zum Auflösen der Metallschicht, wodurch die Metallschicht aufgelöst wird;
- Bestrahlen des Modulators mit Licht, um den Modulator, der den Analyten modifiziert, fotochemisch anzuregen; und
- Detektieren eines fließenden elektrischen Stroms, der von dem fotochemisch angeregten Modulator erzeugt wird.

2. Verfahren nach Anspruch 1, wobei es sich bei der Sonde um eine Nukleinsäure, ein Protein oder ein Peptid handelt.

3. Verfahren nach Anspruch 1 oder 2, wobei das Metall aus Gold, Platin, Silber, Palladium, Nickel, Quecksilber, Rhodium, Ruthenium, Kupfer oder einer Legierung davon ausgewählt ist.

4. Verfahren nach Anspruch 3, wobei es sich bei dem Metall um Gold handelt.

5. Verfahren nach einem der Ansprüche 1 bis 4, wobei die Metallschicht ein Metall umfasst, auf das die Sonde chemisch adsorbiert wird.

6. Verfahren nach einem der Ansprüche 1 bis 5, wobei die Sonde eine Thiolgruppe als Bindungsgruppe für das chemische Adsorbiertwerden auf die Metallschicht aufweist.

7. Verfahren nach einem der Ansprüche 1 bis 6, wobei die Halbleiterschicht eine Schicht, ausgewählt aus Silizium und Germanium oder eine Halbleiterverbindung oder ein organischer Halbleiter ausgewählt aus Titanoxid (TiO₂), Indiumoxid (In₂O₂), Zinnoxid (SnO₂), Zinkoxid (ZnO), Kadmiumselenid (CdSe), Kadmiumsulfid (CdS), Galliumnitrid (GaN) und Titannitrid (TiN) ist.

8. Verfahren nach einem der Ansprüche 1 bis 7, wobei der Gegenelektrodenteil aus einem leitfähigen Material ausgewählt aus Platin, Gold, Silber, Kupfer, leitfähiger Keramik und Metalloxiden gebildet ist.

9. Verfahren nach einem der Ansprüche 1 bis 8, wobei die Lichtquelle, mit der der Modulator bestrahlt wird, aus einem Laser, einer Leuchtdiode (LED), einem anorganischen Elektrolumineszenzelement, einem organischen Elektrolumineszenzelement, einer Weißlichtquelle und einer mit einem optischen Filter versehenen Weißlichtquelle ausgewählt ist.

10. Verfahren nach einem der Ansprüche 1 bis 9, wobei die Metallschicht eine Dicke zwischen 0,2 nm und 2,0 nm aufweist.

## Revendications

1. Procédé de détection d'un analyte modifié avec un modulateur libérant des électrons lors d'une photoexcitation, comprenant :
- le piégeage d'un analyte dans un échantillon en utilisant une puce de test, la puce de test comprenant :
- une partie d'électrode semi-conductrice comportant une couche métallique formée sur une couche semi-conductrice, la couche métallique ayant une épaisseur comprise entre 0,2 et 10 nm, dans lequel quand l'épaisseur de la couche métallique est de 5 nm ou plus, l'adhérence entre la couche métallique et la couche semi-conductrice est améliorée en utilisant une couche semi-conductrice contenant du titane ou du chrome ou en utilisant une couche semi-conductrice soumise à un frittage ;
- une sonde immobilisée sur la couche métallique, la sonde piégeant l'analyte ; et
- une partie de contre-électrode d'une couche conductrice ; dans lequel chaque électrode ne touche pas l'autre électrode ;
- la modification de l'analyte avec un modulateur ;
- l'addition d'un milieu électrolytique comprenant de l'iode en tant qu'électrolyte pour faire passer un courant électrique entre la partie d'électrode semi-conductrice et la partie de contre-électrode et pour dissoudre ladite couche métallique, dissolvant ainsi la couche métallique ;
- l'irradiation du modulateur avec de la lumière pour photoexciter le modulateur modifiant l'analyte ; et
- la détection d'une circulation de courant électrique générée à partir du modulateur photoexcité.

2. Procédé selon la revendication 1, dans lequel la sonde est un acide nucléique, une protéine ou un peptide.

3. Procédé selon la revendication 1 ou 2, dans lequel le métal est choisi parmi l'or, le platine, l'argent, le palladium, le nickel, le mercure, le rhodium, le ruthénium, le cuivre, et un alliage de ceux-ci.

4. Procédé selon la revendication 3, dans lequel le métal est l'or.

5. Procédé selon l'une quelconque des revendications 1 à 4, dans lequel la couche métallique comprend un métal sur lequel la sonde est adsorbée chimiquement.

6. Procédé selon l'une quelconque des revendications 1 à 5, dans lequel la sonde a un groupe thiol en tant que groupe de liaison pour être adsorbée chimiquement sur la couche métallique.

7. Procédé selon l'une quelconque des revendications 1 à 6, dans lequel la couche semi-conductrice est une couche choisie parmi le silicium et le germanium ou un semi-conducteur composé ou un semi-conducteur organique choisi parmi l' oxyde de titane (TiO₂), l'oxyde d'indium (In₂O₂), l'oxyde d'étain (SnO₂), l'oxyde de zinc (ZnO), le séléniure de cadmium (CdSe), le sulfure de cadmium (CdS), le nitrure de gallium (GaN) et le nitrure de titane (TiN).

8. Procédé selon l'une quelconque des revendications 1 à 7, dans lequel la partie de contre-électrode est constituée d'un matériau conducteur choisi parmi le platine, l'or, l'argent, le cuivre, les céramiques conductrices et les oxydes métalliques.

9. Procédé selon l'une quelconque des revendications 1 à 8, dans lequel la source de lumière utilisée pour irradier le modulateur est choisie parmi un laser, une diode électroluminescente (DEL), un élément à électroluminescence inorganique, un élément à électroluminescence organique, une source de lumière blanche, et une source de lumière blanche pourvue d'un filtre optique.

10. Procédé selon l'une quelconque des revendications 1 à 9, dans lequel la couche métallique a une épaisseur comprise entre 0,2 nanomètre et 2,0 nanomètres.
